# EUROPEAN PATENT SPECIFICATION

(11) **EP 3 091 023 B1**
(45) Date of publication and mention of the grant of the patent: **09.11.2022**
(21) Application number: 14876183.6
(22) Date of filing: 07.12.2014
(51) Int. Cl.: C07F 9/30, C08K 5/5313, C08L 67/02, C08L 77/06, C08K 5/02

(54) **ADDITIVE USED IN POLYMER AND PREPARATION METHOD THEREOF**
IN EINEM POLYMER VERWENDETES ADDITIV UND HERSTELLUNGSVERFAHREN DAFÜR
ADDITIF UTILISÉ DANS UN POLYMÈRE ET SON PROCÉDÉ DE PRÉPARATION

(30) Priority: 31.12.2013 CN 201310753931
(43) Date of publication of application: 09.11.2016
(73) Proprietor: Zhuhai Wango Chemical Co., Ltd., Zhuhai, Guangdong 519050 (CN)
(72) Inventor: LI, Jide, Guangzhou Guangdong 510520 (CN); XIA, Shiyong, Guangzhou Guangdong 510520 (CN); WANG, Peng, Guangzhou Guangdong 510520 (CN); CHAI, Shengyong, Guangzhou Guangdong 510520 (CN); CHEN, Lin, Guangzhou Guangdong 510520 (CN); KONG, Lei, Guangzhou Guangdong 510520 (CN); LU, Changli, Guangzhou Guangdong 510520 (CN)
(74) Representative: dompatent von Kreisler Selting Werner - Partnerschaft von Patent- und Rechtsanwälten mbB
(86) International application number: PCT/CN2014/093206
(87) International publication number: WO 2015/101136

(56) References cited:
- CN-A- 1 280 583
- CN-A- 1 660 857
- CN-A- 103 073 575
- CN-A- 103 788 125
- US-A1- 2006 074 157
- US-A1- 2006 084 734
- US-A1- 2007 213 563
- US-A1- 2010 168 289
- US-B1- 6 534 673

## Description

### Technical field

The invention relates to an additive for polymers, especially to dialkylphosphinic salts with trace amount of chlorine and production method thereof.

### Background technology

Dialkylphosphinic salts are widely used as flame retardant and are known to be produced by different methods. For example, Patent DE4430932 discloses that phosphinic salts with two replacement groups are used as flame retardant in polyester. Two patents DE19910232 and US6248921 disclose a production method for phosphinic salts with two replacement groups. US patent US6359171B1 discloses a production method for aluminum dialkylphosphinic. This method firstly uses yellow phosphorous to synthesize monoalkylphosphinate and then uses free radicals to initiate vinylation and hydrolyzing to get acids, which react with aluminum salts to get aluminum dialkylphosphinate flame retardant.

US 2006/084734 A1 describes diorganylphosphinic salts with formula (I) [R₁R₂PO-O]M^{m+} and/or diorganyidiphosphinic salts of the formula (II) [O-POR₁-R₃-POR₂-O]Mₓ^{m+} and/or any of their polymers where R₁ and R₂ are identical or different and are C1-C6-alkyl, linear or branched, and/or aryl; R₃ is C1-C10-alkylene, linear or branched, C6-C10-arylene, -alkylarylene, or -arylalkylene; M is Mg, Ca, Al, Sb, Sn, Ge, Ti, Fe, Zr, Zn, Ce, Bi, Sr, Mn, Li, Na, K, and/or a protonated nitrogen base; m is from 1 to 4; n is from 1 to 4; x is from 1 to 4, wherein the total content of partially water-soluble, ionizable compounds and the water-soluble content of the partially water-soluble, ionizable compounds is from 8200 to 100 ppm, preferably from 7000 to 200 ppm.

US 2010/168289 A1 discloses a composition comprising, based on the total weight of the composition: from 20 to 90 wt. % of a polyester component comprising a poly(butylene terephthalate); from 5 to 35 wt. % of a flame retardant phosphinate of the formula (I) [(R1)(R2)(PO)-O]-mMm+, a flame retardant diphosphinate of the formula (II) [(O-POR1)(R3)(POR2-O)]2-nMm+x, and/or a flame retardant polymer derived from the flame retardant phosphinate of the formula (I) or the flame retardant diphosphinate of the formula (II); from 1 to 25 wt. % of a melamine polyphosphate, melamine cyanurate, melamine pyrophosphate, and/or melamine phosphate; from greater than zero to 50 wt. % of a glass fiber having a non-circular cross-section; and from 0 to 5 wt. % of an additive selected from the group consisting of a mold release agent, an antioxidant, a thermal stabilizer, an antioxidant, and a UV stabilizer; wherein the components have a combined total weight of 100 wt. %.

US 6 534 673 B1 describes a process for preparing salts of dialkylphosphinic acids, which comprisesa) reacting alkylphosphonous and/or hypo- phosphorous acid and/or alkali metal salts thereof with olefins in the presence of a free-radical initiator to give dialkylphosphinic acids and/or alkali metal salts thereof andb) reacting the dialkylphosphinic acids and/or alkali metal salts thereof obtained according to a) with metal compounds of Mg, Ca, Al, Sb, Sn, Ge, Ti, Zn, Fe, Zr, Ce, Bi, Sr, Mn, Li, Na and/or K to give the metal dialkyl phosphinate salts.

US 2006/074157 A1 discloses dialkylphosphinic salts of the formula (I) [R₁R₂PO-O]M^{m+} where R₁, R₂ identical or different, are C1-C6-alkyl, linear or branched M is Mg, Ca, Al, Sb, Sn, Ge, Ti, Fe, Zr, Zn, Ce, Bi, Sr, Mn, Li, Na, K and/or is a protonated nitrogen base; m is from 1 to 4; wherein the telomer content is from 0.01 to 6% by weight. The invention also relates to glare use in flame retardant compositions, and to flame-retardant polymer molding compositions and flame-retardant polymer moldings which comprise these dialkylphosphinic salts.

The use dialkylphosphinic salts as flame retardant to flame-retardant polymers will make the colour of flame-retardant polymers clearly yellow. But after research the invention found that when the powder of flame retardant dialkylphosphinic salts contains trace amount of chloride, the corresponding flame-retardant polymers can achieve much excellent flame retardant performance and mechanical properties, and at the same time, the phenomenon of yellow colour of flame-retardant polymers can be improved obviously.

### Summary of the invention

The object of the invention is to provide an additive for polymers, which can improve polymer article yellowing problem obviously and has efficient flame-retardant effect, through adding trace amount of chlorine into dialkylphosphinic salts.

Another object of the invention is to provide a production method of the additive for polymers aforementioned, which has the characteristics of simple technical process, short reaction period and high overall yield.

The invention can be achieved by the technical solutions below:
An additive for polymers, including
A: dialkylphosphinic salts of the formula (I) 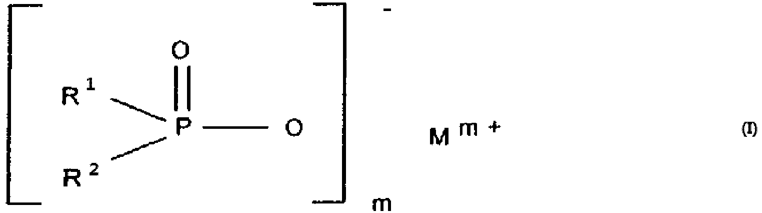 Wherein
   R¹, R² are the same or different, represents C1-C6-alkyl, preferably ethyl, propyl, butyl, hexyl or cyclohexyl;
   M is Mg, Ca, Al, Zn, Fe;
   m is 2-3;
B: chlorine compound, where the weight content of chlorine in total additives is 50-900ppm, preferably 60-800ppm by weight, particularly preferably 70-450ppm by weight, and in particular 100-300ppm by weight, wherein the described chlorine compound is selected from one or several substances of chlorinated olefin, sodium chloride, potassium chloride, aluminum chloride, chlorine gas, hydrochloric acid, sodium hypochlorite, coupling agent containing chlorine, surfactant containing chlorine or PVC.

The acquiring route of the described chlorine in the invention can be choosing reaction raw materials partially containing chlorine, adding chloride during production process, and can also be adding chloride in the product.

The described chlorine compound is selected from one or several substances of chlorinated olefin, sodium chloride, potassium chloride, aluminum chloride, chlorine gas, hydrochloric acid, sodium hypochlorite, coupling agent containing chlorine such as γ-chloropropyltrimethoxysilane coupling agent, surfactant containing chlorine or PVC.

The described chlorinated olefin is one or several substances of 1-cloroethylene, 1,2-dichloroethylene, 3-chloropropylene, 4-chlorobutylene or chlorinated cyclohexene.

The described polymer is polyester (such as PBT, PET), nylon (PA6, PA66, etc.), PPE, TPE, TPU or epoxy resin, preferably PBT, nylon or PPE.

The described efficient flame-retardant dialkylphosphinic salts in the invention can be produced through three methods below.

Method 1- A production method of the additive for polymers, includes procedures below:
a) add phosphinic salt/phosphinic acid and chlorinated olefin into the solvent, and react with olefin in the action of an initiator to get dialkylphosphinic salt/acid solution containing chlorine compound;
b) react dialkylphosphinic salt/acid solution containing chlorine compound with metal compound to get dialkylphosphinic salts containing chlorine compound;
where
the described phosphinic salt is sodium hypophosphite or potassium hypophosphite;
the described metal compound is metal compound of Mg, Ca, Al, Zn and Fe;
the described chlorinated olefin is one or several substances of 1-cloroethylene, 1,2-dichloroethylene, 3-chloropropylene, 4-chlorobutylene or chlorinated cyclohexene.

Method 1 is to add chlorinated olefin during the production process of dialkylphosphinic salts, to get dialkylphosphinic salts containing trace amount of chlorine. The known technology can be used to synthesize dialkylphosphinic salts. For example, the described solvent is cyclohexane and/or cyclohexene; the described initiator is azo-initiator, organic peroxide initiator or inorganic peroxide initiator; the described olefin is one or several mixtures of ethylene, propylene, butylene, iso-butylene or pentene.

Method 2- A production method of the additive for polymers, includes procedures below:
react dialkylphosphinic salt solution with metal compound aqueous solution containing additive I to get the corresponding dialkylphosphinic salts;
where
the described additive I is one or several substances of NaCl, KCI, AlCl₃, chlorine gas, hydrochloric acid, sodium hypochlorite, coupling agent containing chlorine, surfactant containing chlorine;
the described metal compound is metal compound of Mg, Ca, Al, Zn and Fe;
the described dialkylphosphinic salt is sodium dialkylphosphinate or potassium dialkylphosphinate.

Method 3- A production method of the additive for polymers, includes procedures below:
add additive II during filtration process or washing process or drying process of dialkylphosphinic salt to get dialkylphosphinic salts containing chlorine compound;
where
the described additive II is one or several substances of NaCl, KCl, AlCl₃, flame retardant containing chlorine, coupling agent containing chlorine, surfactant containing chlorine or PVC.

The invention also discloses the use as flame retardant of the aforementioned additive for polymers.

The invention also discloses a polymer material containing the aforementioned additive, including 5-20 parts by weight of the additive for polymers, and 50-70 parts by weight of the polymers or their mixtures;
where
the additive for polymers includes:
   A: dialkylphosphinic salts of the formula (I) 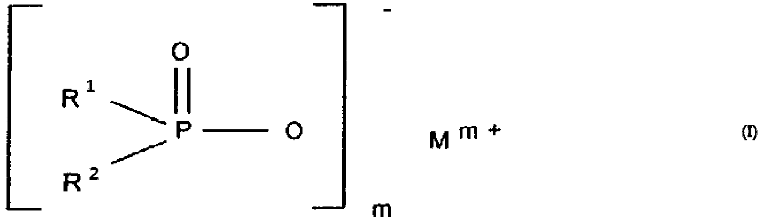 Wherein
      R¹, R² are the sameor different, represents H, C1-C6-alkyl or C6-C18-aryl, preferably ethyl, propyl, butyl, hexyl or cyclohexyl;
      M is Mg, Ca, Al, Zn, Fe;
      m is 2-3;
   B: chlorine compound, where the weight content of chlorine in total additives is 50-900ppm, preferably 60-800ppm by weight, particularly preferably 70-450ppm by weight, and in particular 100-300ppm by weight.

The described chlorine compound is chosen from one or several substances of chlorinated olefin, sodium chloride, potassium chloride, aluminum chloride, chlorine gas, hydrochloric acid, sodium hypochlorite, coupling agent containing chlorine such as γ-chloropropyltrimethoxysilane coupling agent, surfactant containing chlorine or PVC.

The described chlorinated olefin is one or several substances of 1-cloroethylene, 1,2-dichloroethylene, 3-chloropropylene, 4-chlorobutylene or chlorinated cyclohexene.

The described polymer is polyester (such as PBT, PET), nylon (PA6, PA66, etc.), PPE, TPE, TPU or epoxy resin, preferably PBT, nylon or PPE.

The described polymer material in the invention can be added different additives according to the different performance requirement of materials. For example, it also includes 15-30 parts by weight of the glass fiber and 1-5 parts by weight of the other additives.

The invention has beneficial effect below compared to the prior art:
1) Experiments find that the application in polymers as flame retardant of the dialkylphosphinic salts containing trace amount of chlorine produced in the invention can get polymer products with excellent whiteness, and it solves the problem that pure dialkylphosphinic slats as flame retardant make polymer articles yellowing after polymer process modification.
2) Experiments find that dialkylphosphinic salts containing trace amount of chlorine produced in the invention have quite efficient flame retardant performance.
3) The invention can get dialkylphosphinic salts containing trace amount of chlorine by adding chloride during the production process of dialkylphosphinic salts or in the final products, which has the characteristics of simple technical process and low production cost.

### Embodiments

The invention is described further by embodiments below, and the following examples are comparatively good embodiments but embodiments of the invention are not limited by the following examples.

### Comparative example 1:

2120.0g (20mol) sodium hypophosphite , 1320.0g (10mol) 50wt% phosphinic acid, 27.2g (0.3mol% phosphinic acid ion) 1,1-di-(tert-butylperoxy)-3,3,5-trimethylcyclohexane and 3000g water were charged into a pressure reactor, which was evacuated and replaced with nitrogen gas for three times. Evacuate it again and then purge ethylene through a decompressor and control pressure to 2.0MPa. Heat to 87 °C and hold for 5h. Heat to 95 °C and replenish 54.4g (0.6mol% phosphinic acid ion) 1,1-di-(tert-butylperoxy)-3,3,5-trimethylcyclohexane continuously in 5h. Heat to 100 °C and hold for 1h. Cool and evacuate. 8303.0g sodium diethylphosphinate and diethylphosphinic acid mixtures solution were obtained, which were equivalent to 1779.9g ethylene absorptive amount (105.95% of theoretical amount).

³¹P-NMR analysis:
diethylphosphinic acid mole content: 96.7%
ethylbutylphosphinic acid mole content: 1.4%
ethylphosphinic acid mole content: 0.9%
other substances mole content: 1.0%

Take sodium diethylphosphinate and diethylphosphinic acid mixtures solution 830.3g, and add 1600g water, which was neutralized by 30% sodium hydroxide solution. Heat to 90 °C and dropwise add 20% aluminum sulfate octadecahydrate solution prepared by 333.0g (0.5mol) aluminum sulfate octadecahydrate and 1332g water in 1.5h. Hold at 90 °C for 1h. Filtrate and wash with 2000L water for three times to get the filter cake, which was dried to constant weight at 130 °C. 372.2g aluminum diethylphosphinate was obtained and the yield was 95.44%.

Chlorine content: not detectable or less than detection limit.

### Comparative example 2:

2120.0g (20mol) sodium hypophosphite , 1320.0g (10mol) 50% phosphinic acid, 27.2g (0.3mol% phosphinic acid ion) 1,1-di-(tert-butylperoxy)-3,3,5-trimethylcyclohexane and 3000g water were charged into a pressure reactor, which was evacuated and replaced with nitrogen gas for three times. Evacuate it again and purge about 25g chloroethylene and then purge ethylene through a decompressor and control pressure to 2.0MPa. Heat to 87 °C and hold for 5h. Heat to 95 °C and replenish 54.4g (0.6mol% phosphinic acid ion) 1,1-di-(tert-butylperoxy)-3,3,5-trimethylcyclohexane continuously in 5h. Heat to 100 °C and hold for 1h. Cool and evacuate. 8293.8g sodium diethylphosphinate and diethylphosphinic acid mixtures solution were obtained, which were equivalent to 1771.2g ethylene absorptive amount (105.4% of theoretical amount).

³¹P-NMR analysis:
diethylphosphinic acid mole content: 95.9%
ethylbutylphosphinic acid mole content: 2.0%
ethylphosphinic acid mole content: 1.1%
other substances mole content: 1.0%

Take sodium diethylphosphinate and diethylphosphinic acid mixtures solution 830.3g, and add 1600g water, which was neutralized by 30% sodium hydroxide solution. Heat to 90 °C and dropwise add 20% aluminum sulfate octadecahydrate solution prepared by 333.0g (0.5mol) aluminum sulfate octadecahydrate and 1332g water in 1.5h. Hold at 90 °C for 1h. Filtrate and wash with 2000L water for three times to get the filter cake, which was dried to constant weight at 130 °C. 371.3g aluminum diethylphosphinate was obtained and the yield was 95.2%.

Chlorine content: 1158ppm.

### Example 1:

2120.0g (20mol) sodium hypophosphite , 1320.0g (10mol) 50% phosphinic acid, 27.2g (0.3mol% phosphinic acid ion) 1,1-di-(tert-butylperoxy)-3,3,5-trimethylcyclohexane and 3000g water were charged into a pressure reactor, which was evacuated and replaced with nitrogen gas for three times. Evacuate it again and purge about 1g chloroethylene and then purge ethylene through a decompressor and control pressure to 2.5MPa. Heat to 87 °C and hold for 5h. Heat to 95 °C and replenish 54.4g (0.6mol% phosphinic acid ion) 1,1-di-(tert-butylperoxy)-3,3,5-trimethylcyclohexane continuously in 5h. Heat to 100 °C and hold for 1h. Cool and evacuate. 8297.4g sodium diethylphosphinate and diethylphosphinic acid mixtures solution were obtained, which were equivalent to 1774.8g ethylene absorptive amount (105.6% of theoretical amount).

³¹P-NMR analysis:
diethylphosphinic acid mole content: 96.2%
ethylbutylphosphinic acid mole content: 2.2%
ethylphosphinic acid mole content: 1.0%
other substances mole content: 0.6%

Take sodium diethylphosphinate and diethylphosphinic acid mixtures solution 829.7g, and add 1600g water, which was neutralized by 30% sodium hydroxide solution. Heat to 90 °C and dropwise add 20% aluminum sulfate octadecahydrate solution prepared by 333.0g (0.5mol) aluminum sulfate octadecahydrate and 1332g water in 1.5h. Hold at 90 °C for 1h. Filtrate and wash with 2000L water for three times to get the filter cake, which was dried to constant weight at 130 °C. 372.6g aluminum diethylphosphinate was obtained and the yield was 95.5%.

Chlorine content: 53ppm.

### Example 2:

2120.0g (20mol) sodium hypophosphite , 1320.0g (10mol) 50% phosphinic acid, 27.2g (0.3mol% phosphinic acid ion) 1,1-di-(tert-butylperoxy)-3,3,5-trimethylcyclohexane and 3000g water were charged into a pressure reactor, which was evacuated and replaced with nitrogen gas for three times. Evacuate it again and purge about 1.4g chloroethylene and then purge ethylene through a decompressor and control pressure to 2.0MPa. Heat to 87 °C and hold for 5h. Heat to 95 °C and replenish 54.4g (0.6mol% phosphinic acid ion) 1,1-di-(tert-butylperoxy)-3,3,5-trimethylcyclohexane continuously in 5h. Heat to 100 °C and hold for 1h. Cool and evacuate. 8298.9g sodium diethylphosphinate and diethylphosphinic acid mixtures solution were obtained, which were equivalent to 1775.9g ethylene absorptive amount (105.7% of theoretical amount).

³¹P-NMR analysis:
diethylphosphinic acid mole content: 96.1%
ethylbutylphosphinic acid mole content: 2.0%
ethylphosphinic acid mole content: 1.1%
other substances mole content: 0.8%

Take sodium diethylphosphinate and diethylphosphinic acid mixtures solution 829.9g, and add 1600g water, which was neutralized by 30% sodium hydroxide solution. Heat to 90 °C and dropwise add 20% aluminum sulfate octadecahydrate solution prepared by 333.0g (0.5mol) aluminum sulfate octadecahydrate and 1332g water in 1.5h. Hold at 90 °C for 1h. Filtrate and wash with 2000L water for three times to get the filter cake, which was dried to constant weight at 130 °C. 373.9g aluminum diethylphosphinate was obtained and the yield was 95.9%.

Chlorine content: 85ppm.

### Example 3:

2120.0g (20mol) sodium hypophosphite , 1320.0g (10mol) 50% phosphinic acid, 27.2g (0.3mol% phosphinic acid ion) 1,1-di-(tert-butylperoxy)-3,3,5-trimethylcyclohexane and 3000g water were charged into a pressure reactor, which was evacuated and replaced with nitrogen gas for three times. Evacuate it again and purge about 2.3g chloroethylene and then purge ethylene through a decompressor and control pressure to 1.8MPa. Heat to 87 °C and hold for 5h. Heat to 95 °C and replenish 54.4g (0.6mol% phosphinic acid ion) 1,1-di-(tert-butylperoxy)-3,3,5-trimethylcyclohexane continuously in 5h. Heat to 100 °C and hold for 1h. Cool and evacuate. 8296.6g sodium diethylphosphinate and diethylphosphinic acid mixtures solution were obtained, which were equivalent to 1772.1g ethylene absorptive amount (105.5% of theoretical amount).

³¹P-NMR analysis:
diethylphosphinic acid mole content: 96.8%
ethylbutylphosphinic acid mole content: 2.1%
ethylphosphinic acid mole content: 1.2%
other substances mole content: 0.9%

Take sodium diethylphosphinate and diethylphosphinic acid mixtures solution 829.7g, and add 1600g water, which was neutralized by 30% sodium hydroxide solution. Heat to 90 °C and dropwise add 20% aluminum sulfate octadecahydrate solution prepared by 333.0g (0.5mol) aluminum sulfate octadecahydrate and 1332g water in 1.5h. Hold at 90 °C for 1h. Filtrate and wash with 2000L water for three times to get the filter cake, which was dried to constant weight at 130 °C. 373.2g aluminum diethylphosphinate was obtained and the yield was 95.7%.

Chlorine content: 103ppm.

### Example 4:

2120.0g (20mol) sodium hypophosphite , 1320.0g (10mol) 50% phosphinic acid, 27.2g (0.3mol% phosphinic acid ion) 1,1-di-(tert-butylperoxy)-3,3,5-trimethylcyclohexane and 3000g water were charged into a pressure reactor, which was evacuated and replaced with nitrogen gas for three times. Evacuate it again and purge about 11g chloroethylene and then purge ethylene through a decompressor and control pressure to 1.5MPa. Heat to 87 °C and hold for 5h. Heat to 95 °C and replenish 54.4g (0.6mol% phosphinic acid ion) 1,1-di-(tert-butylperoxy)-3,3,5-trimethylcyclohexane continuously in 5h. Heat to 100 °C and hold for 1h. Cool and evacuate. 8303.2g sodium diethylphosphinate and diethylphosphinic acid mixtures solution were obtained, which were equivalent to 1770.6g ethylene absorptive amount (105.4% of theoretical amount).

³¹P-NMR analysis:
diethylphosphinic acid mole content: 95.6%
ethylbutylphosphinic acid mole content: 2.3%
ethylphosphinic acid mole content: 1.3%
other substances mole content: 0.8%

Take sodium diethylphosphinate and diethylphosphinic acid mixtures solution 830.3g, and add 1600g water, which was neutralized by 30% sodium hydroxide solution. Heat to 90 °C and dropwise add 20% aluminum sulfate octadecahydrate solution prepared by 333.0g (0.5mol) aluminum sulfate octadecahydrate and 1332g water in 1.5h. Hold at 90 °C for 1h. Filtrate and wash with 2000L water for three times to get the filter cake, which was dried to constant weight at 130 °C. 374.7g aluminum diethylphosphinate was obtained and the yield was 96.1%.

Chlorine content: 452ppm.

### Example 5:

2120.0g (20mol) sodium hypophosphite , 1320.0g (10mol) 50% phosphinic acid, 27.2g (0.3mol% phosphinic acid ion) 1,1-di-(tert-butylperoxy)-3,3,5-trimethylcyclohexane and 3000g water were charged into a pressure reactor, which was evacuated and replaced with nitrogen gas for three times. Evacuate it again and purge about 18g chloroethylene and then purge ethylene through a decompressor and control pressure to 2.0MPa. Heat to 87 °C and hold for 5h. Heat to 95 °C and replenish 54.4g (0.6mol% phosphinic acid ion) 1,1-di-(tert-butylperoxy)-3,3,5-trimethylcyclohexane continuously in 5h. Heat to 100 °C and hold for 1h. Cool and evacuate. 8308.7g sodium diethylphosphinate and diethylphosphinic acid mixtures solution were obtained, which were equivalent to 1759.1g ethylene absorptive amount (104.7% of theoretical amount).

³¹P-NMR analysis:
diethylphosphinic acid mole content: 95.4%
ethylbutylphosphinic acid mole content: 2.4%
ethylphosphinic acid mole content: 1.2%
other substances mole content: 1.0%

Take sodium diethylphosphinate and diethylphosphinic acid mixtures solution 830.9g, and add 1600g water, which was neutralized by 30% sodium hydroxide solution. Heat to 90 °C and dropwise add 20% aluminum sulfate octadecahydrate solution prepared by 333.0g (0.5mol) aluminum sulfate octadecahydrate and 1332g water in 1.5h. Hold at 90 °C for 1h. Filtrate and wash with 2000L water for three times to get the filter cake, which was dried to constant weight at 130 °C. 375.2g aluminum diethylphosphinate was obtained and the yield was 96.2%.

Chlorine content: 679ppm.

### Example 6:

2120.0g (20mol) sodium hypophosphite , 1320.0g (10mol) 50% phosphinic acid, 27.2g (0.3mol% phosphinic acid ion) 1,1-di-(tert-butylperoxy)-3,3,5-trimethylcyclohexane and 3000g water were charged into a pressure reactor, which was evacuated and replaced with nitrogen gas for three times. Evacuate it again and purge about 27g chloroethylene and then purge ethylene through a decompressor and control pressure to 2.0MPa. Heat to 87 °C and hold for 5h. Heat to 95 °C and replenish 54.4g (0.6mol% phosphinic acid ion) 1,1-di-(tert-butylperoxy)-3,3,5-trimethylcyclohexane continuously in 5h. Heat to 100 °C and hold for 1h. Cool and evacuate. 8314.6g sodium diethylphosphinate and diethylphosphinic acid mixtures solution were obtained, which were equivalent to 1766.0g ethylene absorptive amount (105.1% of theoretical amount).

³¹P-NMR analysis:
diethylphosphinic acid mole content: 96.5%
ethylbutylphosphinic acid mole content: 1.6%
ethylphosphinic acid mole content: 1.0%
other substances mole content: 0.9%

Take sodium diethylphosphinate and diethylphosphinic acid mixtures solution 831.4g, and add 1600g water, which was neutralized by 30% sodium hydroxide solution. Heat to 90 °C and dropwise add 20% aluminum sulfate octadecahydrate solution prepared by 333.0g (0.5mol) aluminum sulfate octadecahydrate and 1332g water in 1.5h. Hold at 90 °C for 1h. Filtrate and wash with 2000L water for three times to get the filter cake, which was dried to constant weight at 130 °C. 375.9g aluminum diethylphosphinate was obtained and the yield was 96.4%.

Chlorine content: 885ppm.

### Example 7:

2120.0g (20mol) sodium hypophosphite , 1320.0g (10mol) 50% phosphinic acid, 5412g (66mol) cyclohexene, 27.2g (0.3mol% phosphinic acid ion) 1,1-di-(tert-butylperoxy)-3,3,5-trimethylcyclohexane and 3000g water were charged into a pressure reactor, which was evacuated and replaced with nitrogen gas for three times. Evacuate it again and purge about 1.5g 3-chloropropylene. Heat to 87 °C and hold for 5h. Heat to 95 °C and replenish 54.4g (0.6mol% phosphinic acid ion) 1,1-di-(tert-butylperoxy)-3,3,5-trimethylcyclohexane continuously in 5h. Heat to 100 °C and hold for 1h. Cool and evacuate. 11933.6g sodium dicyclohexylphosphinate and dicyclohexylphosphinic acid mixtures solution were obtained.

³¹P-NMR analysis:
dicyclohexylphosphinic acid mole content: 93.8%
cyclohexylphosphinic acid mole content: 3.4%
other substances mole content: 2.8%

Take sodium dicyclohexylphosphinate and dicyclohexylphosphinic acid mixtures solution 1193.4g, and add 1600g water, which was neutralized by 30% sodium hydroxide solution. Heat to 90 °C and dropwise add 15% anhydrous magnesium sulfate solution prepared by 180.6g (1.5mol) anhydrous magnesium sulfate and 682.3g water in 1.5h. Hold at 90 °C for 1h. Filtrate and wash with 1000L water for three times to get the filter cake, which was dried to constant weight at 130 °C. 561.1g magnesium dicyclohexylphosphinate was obtained and the yield was 77.61%.

Chlorine content: 74ppm.

### Example 8:

2120.0g (20mol) sodium hypophosphite , 1320.0g (10mol) 50% phosphinic acid, 27.2g (0.3mol% phosphinic acid ion) 1,1-di-(tert-butylperoxy)-3,3,5-trimethylcyclohexane and 3000g water were charged into a pressure reactor, which was evacuated and replaced with nitrogen gas for three times. Evacuate it again and purge about 2.0g 4-chloro-1-butylene and then purge propylene through a decompressor and control pressure to 1.5MPa. Heat to 87 °C and hold for 6h. Heat to 95 °C and replenish 54.4g (0.6mol% phosphinic acid ion) 1,1-di-(tert-butylperoxy)-3,3,5-trimethylcyclohexane continuously in 5h. Heat to 100 °C and hold for 1h. Cool and evacuate. 9146.4g sodium dipropylphosphinate and dipropylphosphinic acid mixtures solution were obtained, which were equivalent to 2623.3g propylene absorptive amount (103.10% of theoretical amount).

³¹P-NMR analysis:
dipropylphosphinic acid mole content: 95.0%
propylphosphinic acid mole content: 3.2%
other substances mole content: 1.8%

Take sodium dipropylphosphinate and dipropylphosphinic acid mixtures solution 914.6g, and add 1750g water, which was neutralized by 30% sodium hydroxide solution. Heat to 90 °C and dropwise add 15% anhydrous ferric sulfate solution prepared by 200.0 g(0.5mol) anhydrous ferric sulfate and 1133.2g water in 1.5h. Hold at 90 °C for 1h. Filtrate and wash with 1500L water for three times to get the filter cake, which was dried to constant weight at 130 °C. 382.3g ferric dipropylphosphinate was obtained and the yield was 91.25%.

Chlorine content: 55ppm.

### Example 9:

2120.0g (20mol) sodium hypophosphite , 1320.0g (10mol) 50% phosphinic acid, 27.2g (0.3mol% phosphinic acid ion) 1,1-Di-(tert-butylperoxy)-3,3,5-trimethylcyclohexane and 3000g water were charged into a pressure reactor, which was evacuated and replaced with nitrogen gas for three times. Evacuate it again and purge about 5.0g 3-chlorocyclohexene and then purge butylene through a decompressor and control pressure to 1.0MPa. Heat to 87 °C and hold for 5h. Heat to 95 °C and replenish 54.4g (0.6mol% phosphinic acid ion) 1,1-Di-(tert-butylperoxy)-3,3,5-trimethylcyclohexane continuously in 5h. Heat to 100 °C and hold for 1h. Cool and evacuate. 8298.1g sodium dibutylphosphinate and dibutylphosphinic acid mixtures solution were obtained, which were equivalent to 1775.0g butylene absorptive amount (105.65% of theoretical amount).

³¹P-NMR analysis:
dibutylphosphinic acid mole content: 95.4%
butylphosphinic acid mole content: 2.9%
other substances mole content: 1.7%

Take sodium dibutylphosphinate and dibutylphosphinic acid mixtures solution 829.8g, and add 1600g water, which was neutralized by 30% sodium hydroxide solution. Heat to 90 °C and dropwise add 25% zinc sulfate heptahydrate solution prepared by 431.4g (1.5mol) zinc sulfate heptahydrate and 1294.2g water in 1.5h. Hold at 90 °C for 1h. Filtrate and wash with 2000L water for three times to get the filter cake, which was dried to constant weight at 130 °C. 583.7g zinc dibutylphosphinate was obtained and the yield was 92.87%.

Chlorine content: 93ppm.

### Example 10:

277.7g (0.417mol) aluminum sulfate octadecahydrate, 22.4g (0.093mol) aluminum chloride hexahydrate and 900g water was prepared a solution, which was added dropwise in 1h to 2000g (2.78mol) 20wt% sodium diethylphosphinate preheated to 90 °C. Hold at 90 °C for 1h. Filtrate and wash with 600ml water for four times to get the filter cake, which was dried to constant weight at 130 °C. 353.7g aluminum diethylphosphinate was obtained and the yield was 97.87%.

Chlorine content: 74ppm.

### Example 11:

223.7g (0.927mol) aluminum chloride hexahydrate and 900g water was prepared a solution, which was added dropwise in 1h to 2000g (2.78mol) 20wt% sodium diethylphosphinate preheated to 90 °C. Hold at 90 °C for 1h. Filtrate and wash with 600ml water for four times to get the filter cake, which was dried to constant weight at 130 °C. 353.2g aluminum diethylphosphinate was obtained and the yield was 97.73%.

Chlorine content: 873ppm.

### Example 12:

277.7g (0.463mol) aluminum sulfate octadecahydrate, 16.26g (0.28mol) sodium chloride and 900g water was prepared a solution, which was added dropwise in 1h to 2000g (2.78mol) 20wt% sodium diethylphosphinate preheated to 90 °C. Hold at 90 °C for 1h. Filtrate and wash with 600ml water for four times to get the filter cake, which was dried to constant weight at 130 °C. 351.0g aluminum diethylphosphinate was obtained and the yield was 97.11%.

Chlorine content: 329ppm.

### Example 13:

277.7g (0.463mol) aluminum sulfate octadecahydrate, 62.58g (0.84mol) potassium chloride and 900g water was prepared a solution, which was added dropwise in 1h to 2000g (2.78mol) 20wt% sodium diethylphosphinate preheated to 90 °C. Hold at 90 °C for 1h. Filtrate and wash with 600ml water for four times to get the filter cake, which was dried to constant weight at 130 °C. 353.2g aluminum diethylphosphinate was obtained and the yield was 97.73%.

Chlorine content: 522ppm.

### Example 14:

Take 830.3g sodium diethylphosphinate and diethylphosphinic acid mixtures solution prepared as comparative example 1, and add 1600g water, which was neutralized by 30% sodium hydroxide. Heat to 90 °C and dropwise add 20% aluminum sulfate octadecahydrate solution prepared by 333.0g (0.5mol) aluminum sulfate octadecahydrate and 1332g water in 1.5h. Hold at 90°C for 1h. Filtrate and wash with 2000L water containing 1wt% γ-chloropropyltrimethoxysilane coupling agent for three times to get the filter cake, which was dried to constant weight at 130 °C. 373.1g aluminum diethylphosphinate was obtained.

Chlorine content: 98ppm.

### Example 15:

Take 830.3g sodium diethylphosphinate and diethylphosphinic acid mixtures solution prepared as comparative example 1, and add 1600g water, which was neutralized by 30% sodium hydroxide. Heat to 90 °C and dropwise add 20% aluminum sulfate octadecahydrate solution prepared by 333.0g (0.5mol) aluminum sulfate octadecahydrate and 1332g water in 1.5h. Hold at 90 °C for 1h. Filtrate and wash with 2000L water containing 2wt% y-chloropropyltriethoxysilane coupling agent for three times to get the filter cake, which was dried to constant weight at 130 °C. 373.1g aluminum diethylphosphinate was obtained.

Chlorine content: 105ppm.

### Example 16-32:

Dialkylphsphinic salts obtained from examples 1-15 (A1-A15) and comparative examples 1-2 (B1-B2) were mixed with PBT, glass fiber and the additive by weight ratio 9:50:30:11 at 230-260 °C, and extrude from a twin-screw extruder to get flame-retardant thermoplastic polymer moulding materials. Prepare samples to test their combustion performance and mechanical properties, and the tested results are listed in Table 1:

**Table 1 The combustion performance and mechanical properties parameters of flame-retardant PBT material produced from dialkylphosphinic salts**

| 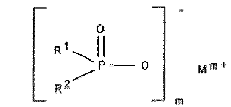 | | | | | GB Tensile strength (Mpa) | GB Flexural strength (Mpa) | Deflection (mm) | UL94 flammab ility 1.6mm | UL94 flammabil ity 2.5mm | colour | | |
|---|---|---|---|---|---|---|---|---|---|---|---|---|
| example | | name | chlorine content | possible substances introducing chlorine ion | | | | | | L | a | b |
| 16 | B1 | aluminum diethylphosphinate | not detectable | | 106 | 169 | 4.1 | V1 | V0 | 80.86 | 0.89 | 1.96 |
| 17 | B2 | aluminum diethylphosphinate | 1158ppm | | 114 | 172 | 4.2 | V0 | V0 | 85.66 | 1.23 | 6.76 |
| 18 | A1 | aluminum diethylphosphinate | 53ppm | aluminum chlorinated-ethyl ethyl phosphinate | 115 | 173 | 4.4 | V0 | V0 | 84.55 | 1.01 | 5.56 |
| 19 | A2 | aluminum diethylphosphinate | 85 ppm | aluminum chlorinated-ethyl ethyl phosphinate | 117 | 174 | 4.3 | V0 | V0 | 85.66 | 1.09 | 5.93 |
| 20 | A3 | aluminum diethylphosphinate | 103 ppm | aluminum chlorinated-ethyl ethyl phosphinate | 119 | 178 | 4.2 | V0 | V0 | 85.69 | 1.13 | 6.27 |
| 21 | A4 | aluminum diethylphosphinate | 452 ppm | aluminum chlorinated-ethyl ethyl phosphinate | 113 | 173 | 4.3 | V0 | V0 | 86.52 | 1.34 | 6.21 |
| 22 | A5 | aluminum diethylphosphinate | 679 ppm | aluminum chlorinated-ethyl ethyl phosphinate | 115 | 177 | 4.0 | V0 | V0 | 86.90 | 1.24 | 6.41 |
| 23 | A6 | aluminum diethylphosphinate | 885ppm | aluminum chlorinated-ethyl ethyl phosphinate | 116 | 178 | 4.2 | V0 | V0 | 87.81 | 1.20 | 6.20 |
| 24 | A7 | magnesium dicyclohexylphosphi nate | 74ppm | magnesium 3-chloropropyl cyclohexyl phosphinate | 102 | 163 | 4.0 | V2 | V0 | 85.42 | 1.42 | 5.85 |
| 25 | A8 | ferric dipropylphosphinat e | 55ppm | ferric 4-chlorobutyl propyl phosphinate | 115 | 178 | 4.0 | V0 | V0 | 84.33 | 1.00 | 5.77 |
| 26 | A9 | zinc dibutylphosphinate | 93ppm | zinc chlorinated-cyclohexyl butyl phosphinate | 107 | 175 | 4.0 | V0 | V0 | 86.16 | 1.16 | 5.74 |
| 27 | A10 | aluminum diethylphosphinate | 74ppm | aluminum chloride | 106 | 173 | 4.1 | V0 | V0 | 85.24 | 1.05 | 5.67 |
| 28 | A11 | aluminum diethylphosphinate | 522ppm | aluminum chloride | 114 | 170 | 4.1 | V0 | V0 | 86.62 | 0.87 | 6.01 |
| 29 | A12 | aluminum diethylphosphinate | 329ppm | sodium chloride | 113 | 172 | 4.3 | V0 | V0 | 85.12 | 1.00 | 5.79 |
| 30 | A13 | aluminum diethylphosphinate | 522ppm | potassium chloride | 119 | 179 | 4.3 | V0 | V0 | 86.43 | 0.85 | 5.72 |
| | | | | | | | | | | | | |
| 31 | A14 | aluminum diethylphosphinate | 98ppm | Y-chloropropyltrimethoxysi lane coupling agent | 116 | 172 | 4.2 | V0 | V0 | 85.75 | 0.92 | 5.64 |
| 32 | A15 | aluminum diethylphosphinate | 105ppm | Y-chloropropyltriethoxysila ne coupling agent | 121 | 164 | 4.0 | V0 | V0 | 86.02 | 0.92 | 5.66 |
| | | | | | | | | | | | | |
| | | | | | | | | | | | | |

### Examples 33-49

Dialkylphsphinic salts obtained from examples 1-15 (A1-A15) and comparative examples 1-2 (B1-B2) were mixed with PA66, glass fiber and the additive by weight ratio 11:52:30:7 at 260-280 °C, and extrude from a twin-screw extruder to get flame-retardant thermoplastic polymer moulding materials. Prepare samples to test their combustion performance and mechanical properties, and the tested results are listed in Table 2:

**Table 2 The combustion performance and mechanical properties parameters of flame-retardant PA66 material produced from dialkylphosphinic salts**

| 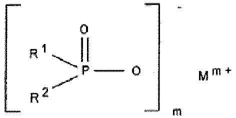 | | | | | GB Tensile strength (Mpa) | GB Flexural strength (Mpa) | Deflectio n (mm) | UL94 flammabilit y 1.6mm | UL94 flammabili ty 2.5mm | colour | | |
|---|---|---|---|---|---|---|---|---|---|---|---|---|
| exam ple | | name | chlorine content | possible substances introducing chlorine ion | | | | | | L | a | b |
| 33 | B1 | aluminum diethylphosphinate | not detectab le | | 115 | 168 | 5.1 | V1 | V0 | 72.10 | -5.15 | -2.11 |
| 34 | B2 | aluminum diethylphosphinate | 1158pp m | | 123 | 182 | 5.6 | V0 | V0 | 75.16 | -2.13 | 0.19 |
| 35 | A1 | aluminum diethylphosphinate | 53ppm | aluminum chlorinated-ethyl ethyl phosphinate | 125 | 180 | 5.8 | V0 | V0 | 73.21 | -3.08 | -1.19 |
| 36 | A2 | aluminum diethylphosphinate | 85 ppm | aluminum chlorinated-ethyl ethyl phosphinate | 122 | 182 | 5.6 | V0 | V0 | 73.69 | -2.98 | -1.09 |
| 37 | A3 | aluminum diethylphosphinate | 103 ppm | aluminum chlorinated-ethyl ethyl phosphinate | 127 | 177 | 5.4 | V0 | V0 | 73.70 | -3.18 | -1.11 |
| 38 | A4 | aluminum diethylphosphinate | 452 ppm | aluminum chlorinated-ethyl ethyl phosphinate | 121 | 179 | 5.4 | V0 | V0 | 74.85 | -3.44 | 0.25 |
| 39 | A5 | aluminum diethylphosphinate | 679 ppm | aluminum chlorinated-ethyl ethyl phosphinate | 126 | 175 | 5.6 | V0 | V0 | 75.54 | -2.80 | 0.30 |
| 40 | A6 | aluminum diethylphosphinate | 885ppm | aluminum chlorinated-ethyl ethyl phosphinate | 121 | 172 | 5.5 | V0 | V0 | 75.78 | -3.14 | 0.22 |
| 41 | A7 | magnesium dicyclohexylphosphinate | 74ppm | magnesium 3-chloropropyl cyclohexyl phosphinate | 127 | 182 | 5.7 | V0 | V0 | 73.68 | -2.25 | -1.25 |
| 42 | A8 | ferric dipropylphosphinate | 55ppm | ferric 4-chlorobutyl propyl phosphinate | 119 | 170 | 5.5 | V0 | V0 | 73.44 | -2.29 | -1.40 |
| 43 | A9 | zinc dibutylphosphinate | 93ppm | zinc chlorinated-cyclohexyl butyl phosphinate | 128 | 184 | 5.8 | V0 | V0 | 74.06 | -2.78 | -1.35 |
| 44 | A10 | aluminum diethylphosphinate | 74ppm | aluminum chloride | 128 | 177 | 5.9 | V0 | V0 | 73.77 | -2.98 | -1.66 |
| 45 | A11 | aluminum diethylphosphinate | 522ppm | aluminum chloride | 126 | 179 | 5.7 | V0 | V0 | 75.29 | -3.05 | 0.24 |
| 46 | A12 | aluminum diethylphosphinate | 329ppm | sodium chloride | 125 | 182 | 5.8 | V0 | V0 | 75.02 | -2.95 | 0.35 |
| 47 | A13 | aluminum diethylphosphinate | 522ppm | potassium chloride | 120 | 185 | 5.4 | V0 | V0 | 75.66 | -1.55 | 0.30 |
| | | | | | | | | | | | | |
| 48 | A14 | aluminum diethylphosphinate | 98ppm | Y-chloropropyltrimetho xysilane coupling agent | 122 | 179 | 5.5 | V0 | V0 | 74.02 | -2.95 | -1.35 |
| 49 | A15 | aluminum diethylphosphinate | 105ppm | Y-chloropropyltriethoxy silane coupling agent | 128 | 175 | 5.8 | V0 | V0 | 74.66 | -2.55 | -1.30 |
| | | | | | | | | | | | | |
| | | | | | | | | | | | | |

It can be observed from test results of the aforementioned Table 1-2 that the polymer materials produced from dialkylphosphinic salts containing trace amount of chlorine produced in the invention can improve the problem of polymer article yellowing obviously and has excellent flame retardant performance and mechanical properties.

Performance tests proceed according to these standards below:
Tensile strength: GB1040-1992 Test methods of plastic tensile performances;
Flexural strength: GB9341-2000 Test methods of plastic flexural performances;
Deflection: GB9341-2000 Test methods of plastic flexural performances;
Combustion performance: UL94 Tests of plastic combustion performances;
Colour test standard:
   Colour test proceeds on Color-Eye-7000A colour photometer (Gretag Macbeth company), and the specific test method is:
   1. Injection mould the resin to a colour disk about 2mm thick;
   2. Set the test diameter and other parameters of the Lab tester;
   3. Put the colour disk on the test window, and click "test" button to conduct Lab test. The system can show the corresponding Lab;

For PBT natural colour material, the acceptable Lab is L: 85.50, a:1.35, b: 5.60. The higher the "L", the lighter the colour, and the colour blending can be more convenient.

Chlorine content test standard: prEN 14582-2002 Test method of halogen.

## Claims

1. An additive for polymers, including
A: dialkylphosphinic salts of the formula (I) 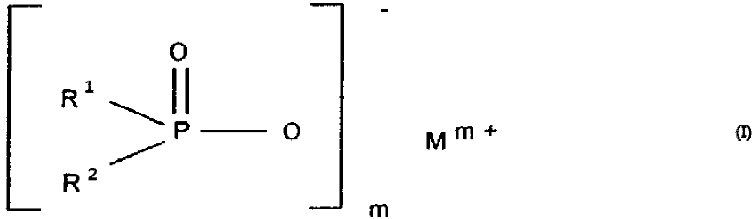 wherein
R¹, R² are the same or different, represents C1-C6-alkyl M is Mg, Ca, Al, Zn, Fe;
m is 2-3;
B: **chlorine compound,** where the weight content of chlorine in total additives is 50-900ppm, wherein the described **chlorine compound** is selected from the group consisting of one or several substances of chlorinated olefin, sodium chloride, potassium chloride, aluminum chloride, chlorine gas, hydrochloric acid, sodium hypochlorite, coupling agent containing chlorine, surfactant containing chlorine or PVC.

2. The additive for polymers as claimed in claim 1, **characterized in that** R¹, R² are ethyl, propyl, butyl, hexyl or cyclohexyl.

3. The additive for polymers as claimed in claim 1, **characterized in that** the weight content of the described chlorine in total additives is 60-800ppm, preferably 70-450ppm by weight, particularly preferably 100-300ppm by weight.

4. The additive for polymers as claimed in claim 1, **characterized in that** the described chlorinated olefin is one or several substances of 1-cloroethylene, 1,2-dichloroethylene, 3-chloropropylene, 4-chlorobutylene or chlorinated cycl ohexene.

5. The additive for polymers as claimed in claim 1, **characterized in that** the described polymer is polyester, nylon, PPE, TPE, TPU or epoxy resin, preferably polyester, nylon or PPE.

6. A production method of the additive for polymers as claimed in claim 1, **characterized in that** it includes procedures below:
a) add phosphinic salt/phosphinic acid and chlorinated olefin into the solvent, and react with olefin in the action of an initiator to get dialkylphosphinic salt/acid solution containing **chlorine compound;**
b) react dialkylphosphinic salt/acid solution containing **chlorine compound** with metal compound to get dialkylphosphinic salts containing **chlorine compound;**
where
the described phosphinic salt is sodium hypophosphite or potassium hypophosphite;
the described metal compound is metal compound of Mg, Ca, Al, Zn and Fe;
the described chlorinated olefin is one or several substances of 1-cloroethylene, 1,2-dichloroethylene, 3-chloropropylene, 4-chlorobutylene or chlorinated cycl ohexene.

7. A production method of the additive for polymers as claimed in claim 6, **characterized in that** the described solvent is cyclohexane and/or cyclohexene; the described initiator is azo-initiator, organic peroxide initiator or inorganic peroxide initiator; the described olefin is one or several mixtures of ethylene, propylene, butylene, iso-butylene or pentene.

8. A production method of the additive for polymers as claimed in claim 1, **characterized in that** it includes procedures below:
react dialkylphosphinic salt solution with metal compound aqueous solution containing additive I to get the corresponding dialkylphosphinic salts;
where
the described additive I is one or several substances of NaCl, KCI, AlCl₃, chlorine gas, hydrochloric acid, sodium hypochlorite, coupling agent containing chlorine, surfactant containing chlorine;
the described metal compound is metal compound of Mg, Ca, Al, Zn and Fe;
the described dialkylphophinic salt is sodium dialkylphosphinate or potassium dialkylphosphinate.

9. A production method of the additive for polymers as claimed in claim 1, **characterized in that** it includes procedures below:
add additive II during filtration process or washing process or drying process of dialkylphosphinic salt to get dialkylphosphinic salts containing **chlorine compound;**
where
the described additive II is one or several substances of NaCl, KCI, AlCl₃, flame retardant containing chlorine, coupling agent containing chlorine, surfactant containing chlorine or PVC.

10. The use as flame retardant of the additive for polymers as claimed in claim 1.

11. A polymer material containing the additive as claimed in claim 1, including 5-20 parts by weight of the additive for polymers, and 50-70 parts by weight of the polymers or their mixtures;
wherein
the additive for polymers includes:
A: dialkylphosphinic salts of the formula (I) 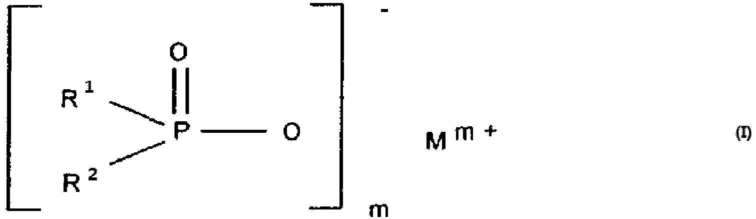 wherein
R¹, R² are the same or different, represents H, C1-C6-alkyl or C6-C18-aryl; M is Mg, Ca, Al, Zn, Fe;
m is 2-3;
B: **chlorine compound,** where the weight content of chlorine in total additives is 50-900ppm, wherein the described **chlorine compound** is selected from the group consisting of one or several substances of chlorinated olefin, sodium chloride, potassium chloride, aluminum chloride, chlorine gas, hydrochloric acid, sodium hypochlorite, coupling agent containing chlorine, surfactant containing chlorine or PVC.

12. The polymer material as claimed in claim 11, **characterized in that** R¹, R² are ethyl, propyl, butyl, hexyl or cyclohexyl.

13. The polymer material as claimed in claim 11, **characterized in that** the weight content of the described chlorine in total additives is 60-800ppm, preferably 70-450ppm by weight, particularly preferably 100-300ppm by weight.

14. The polymer material as claimed in claim 11, **characterized in that** the described chlorinated olefin is one or several substances of 1-cloroethylene, 1,2-dichloroethylene, 3-chloropropylene, 4-chlorobutylene or chlorinated cycl ohexene.

15. The polymer material as claimed in claim 11, **characterized in that** the described polymer is polyester, nylon, PPE, TPE, TPU or epoxy resin, preferably polyester, nylon or PPE.

16. The polymer material as claimed in claim 11, **characterized in that** it also includes 15-30 parts by weight of the glass fiber and 1-5 parts by weight of the other additives.

## Patentansprüche

1. Additiv für Polymere, umfassend
A: Dialkylphosphinsäuresalze der Formel (I) 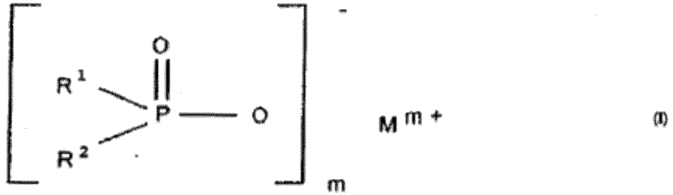 wobei
R¹, R² gleich oder verschieden sind und für C₁-C₆-Alkyl stehen;
M = Mg, Ca, Al, Zn, Fe ist;
m = 2-3 ist;
B: eine Chlorverbindung, wobei der Gewichtsanteil von Chlor in den gesamten Additiven 50-900 ppm beträgt, wobei die genannte Chlorverbindung aus der Gruppe ausgewählt ist, die aus einer oder mehreren der Substanzen chloriertes Olefin, Natriumchlorid, Kaliumchlorid, Aluminiumchlorid, Chlorgas, Chlorwasserstoffsäure, Natriumhypochlorit, chlorhaltiges Kopplungsmittel, chlorhaltiges Tensid oder PVC besteht.

2. Additiv für Polymere gemäß Anspruch 1, **dadurch gekennzeichnet, dass** es sich bei R¹, R² um Ethyl, Propyl, Butyl, Hexyl oder Cyclohexyl handelt.

3. Additiv für Polymere gemäß Anspruch 1, **dadurch gekennzeichnet, dass** der Gewichtsanteil des genannten Chlors in den gesamten Additiven 60-800 ppm, vorzugsweise 70-450 ppm, besonders bevorzugt 100-300 ppm, jeweils bezogen auf das Gewicht, beträgt.

4. Additiv für Polymere gemäß Anspruch 1, **dadurch gekennzeichnet, dass** es sich bei dem genannten chlorierten Olefin um eine oder mehrere der Substanzen 1-Chlorethylen, 1,2-Dichlorethylen, 3-Chlorpropylen, 4-Chlorbutylen oder chloriertes Cyclohexen handelt.

5. Additiv für Polymere gemäß Anspruch 1, **dadurch gekennzeichnet, dass** es sich bei dem genannten Polymer um Polyester, Nylon, PPE, TPE, TPU oder Epoxidharz, vorzugsweise Polyester, Nylon oder PPE, handelt.

6. Herstellungsverfahren für das Additiv für Polymere gemäß Anspruch 1, **dadurch gekennzeichnet, dass** es die folgenden Arbeitsschritte umfasst:
a) Hinzufügen von Phosphinsäuresalz/Phosphinsäure und chloriertem Olefin zu dem Lösungsmittel und Umsetzen mit Olefin unter der Wirkung eines Initiator, wobei man eine Lösung von Dialkylphosphinsäuresalz/-säure erhält, die eine Chlorverbindung enthält;
b) Umsetzen der Lösung von Dialkylphosphinsäuresalz/-säure, die die Chlorverbindung enthält, mit einer Metallverbindung unter Bildung von Dialkylphosphinsäuresalzen, die die Chlorverbindung enthalten;
wobei
es sich bei dem genannten Phosphinsäuresalz um Natriumhypophosphit oder Kaliumhypophosphit handelt;
die genannte Metallverbindung eine Metallverbindung von Mg, Ca, Al, Zn und Fe ist;
es sich bei dem genannten chlorierten Olefin um eine oder mehrere der Substanzen 1-Chlorethylen, 1,2-Dichlorethylen, 3-Chlorpropylen, 4-Chlorbutylen oder chloriertes Cyclohexen handelt.

7. Herstellungsverfahren für das Additiv für Polymere gemäß Anspruch 6, **dadurch gekennzeichnet, dass** es sich bei dem genannten Lösungsmittel um Cyclohexan und/oder Cyclohexen handelt; der genannte Initiator ein Azoinitiator, ein Initiator in Form eines organischen Peroxids oder ein Initiator in Form eines anorganischen Peroxids ist; es sich bei dem genannten Olefin um eines oder mehrere Gemische von Ethylen, Propylen, Butylen, Isobutylen oder Penten handelt.

8. Herstellungsverfahren für das Additiv für Polymere gemäß Anspruch 1, **dadurch gekennzeichnet, dass** es die folgenden Arbeitsschritte umfasst:
Umsetzen einer Dialkylphosphinsäuresalzlösung mit einer wässrigen Lösung einer Metallverbindung, die Additiv I enthält, unter Bildung der entsprechenden Dialkylphosphinsäuresalze;
wobei
es sich bei dem genannten Additiv I um eine oder mehrere der Substanzen NaCl, KCl, AlCl₃, Chlorgas, Chlorwasserstoffsäure, Natriumhypochlorit, chlorhaltiges Kopplungsmittel, chlorhaltiges Tensid handelt;
die genannte Metallverbindung eine Metallverbindung von Mg, Ca, Al, Zn und Fe ist;
es sich bei dem genannten Dialkylphosphinsäuresalz um Natriumdialkylphosphinat oder Kaliumdialkylphosphinat handelt.

9. Herstellungsverfahren für das Additiv für Polymere gemäß Anspruch 1, **dadurch gekennzeichnet, dass** es die folgenden Arbeitsschritte umfasst:
Hinzufügen von Additiv II während eines Filtrationsvorgangs oder Waschvorgangs oder Trocknungsvorgangs von Dialkylphosphinsäuresalz unter Bildung von Dialkylphosphinsäuresalzen, die eine Chlorverbindung enthalten;
wobei
es sich bei dem genannten Additiv II um eine oder mehrere der Substanzen NaCl, KCl, AlCl₃, chlorhaltiges Flammschutzmittel, chlorhaltiges Kopplungsmittel, chlorhaltiges Tensid oder PVC handelt.

10. Verwendung des Additivs für Polymere gemäß Anspruch 1 als Flammschutzmittel.

11. Polymermaterial, das das Additiv gemäß Anspruch 1 enthält, umfassend 5-20 Gewichtsteile des Additivs für Polymere und 50-70 Gewichtsteile der Polymere oder ihrer Gemische;
wobei
das Additiv für Polymere Folgendes umfasst:
A: Dialkylphosphinsäuresalze der Formel (I) 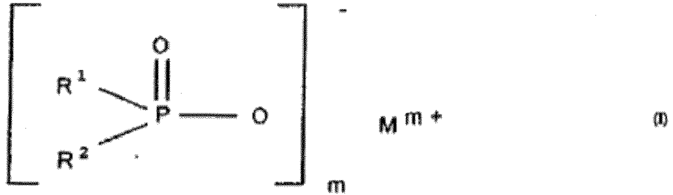 wobei
R¹, R² gleich oder verschieden sind und für H, C₁-C₆-Alkyl oder C₆-C₁₈-Aryl stehen;
M = Mg, Ca, Al, Zn, Fe ist;
m = 2-3 ist;
B: Chlorverbindung, wobei der Gewichtsanteil von Chlor in den gesamten Additiven 50-900 ppm beträgt, wobei die genannte Chlorverbindung aus der Gruppe ausgewählt ist, die aus einer oder mehreren der Substanzen chloriertes Olefin, Natriumchlorid, Kaliumchlorid, Aluminiumchlorid, Chlorgas, Chlorwasserstoffsäure, Natriumhypochlorit, chlorhaltiges Kopplungsmittel, chlorhaltiges Tensid oder PVC besteht.

12. Polymermaterial gemäß Anspruch 11, **dadurch gekennzeichnet, dass** es sich bei R¹, R² um Ethyl, Propyl, Butyl, Hexyl oder Cyclohexyl handelt.

13. Polymermaterial gemäß Anspruch 11, **dadurch gekennzeichnet, dass** der Gewichtsanteil des genannten Chlors in den gesamten Additiven 60-800 ppm, vorzugsweise 70-450 ppm, besonders bevorzugt 100-300 ppm, jeweils bezogen auf das Gewicht, beträgt.

14. Polymermaterial gemäß Anspruch 11, **dadurch gekennzeichnet, dass** es sich bei dem genannten chlorierten Olefin um eine oder mehrere der Substanzen 1-Chlorethylen, 1,2-Dichlorethylen, 3-Chlorpropylen, 4-Chlorbutylen oder chloriertes Cyclohexen handelt.

15. Polymermaterial gemäß Anspruch 11, **dadurch gekennzeichnet, dass** es sich bei dem genannten Polymer um Polyester, Nylon, PPE, TPE, TPU oder Epoxidharz, vorzugsweise Polyester, Nylon oder PPE, handelt.

16. Polymermaterial gemäß Anspruch 11, **dadurch gekennzeichnet, dass** es auch 15-30 Gewichtsteile Glasfaser und 1-5 Gewichtsteile der anderen Additive umfasst.

## Revendications

1. Additif pour polymères, comprenant
A: sels dialkylphosphiniques répondant à la formule (I) 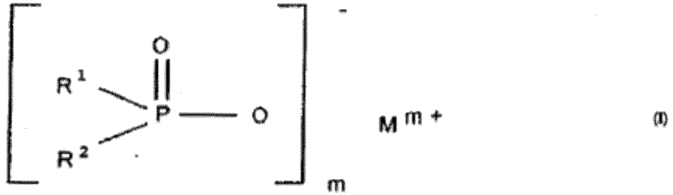 où
R¹, R² sont identiques ou différents et représentent C₁-C₆-alkyle;
M est Mg, Ca, Al, Zn, Fe;
m est 2-3;
B: composé de chlore, dans lequel la proportion pondérale de chlore dans les additifs totaux est 50-900 ppm, dans lequel ledit composé de chlore est choisi dans le groupe consistant en une ou plusieurs des substances oléfine chloré, chlorure de sodium, chlorure de potassium, chlorure d'aluminium, chlore gazeux, acide chlorhydrique, hypochlorite de sodium, agent de couplage contenant du chlore, tensioactif contenant du chlore, ou PVC.

2. Additif pour polymères selon la revendication 1, **caractérisé en ce que** R¹, R² sont éthyle, propyle, butyle, hexyle ou cyclohexyle.

3. Additif pour polymères selon la revendication 1, **caractérisé en ce que** la proportion pondérale dudit chlore dans les additifs totaux est 60-800 ppm, de préférence 70-450 ppm en poids, de préférence encore 100-300 ppm en poids.

4. Additif pour polymères selon la revendication 1, **caractérisé en ce que** ledit oléfine chloré est une ou plusieurs substances parmi 1-chloroéthylène, 1,2-dichloroéthylène, 3-chloropropylène, 4-chlorobutylène, ou cyclohexène chloré.

5. Additif pour polymères selon la revendication 1, **caractérisé en ce que** ledit polymère est polyester, nylon, PPE, TPE, TPU ou résine époxy, de préférence polyester, nylon ou PPE.

6. Procédé de production de l'additif pour polymères selon la revendication 1, **caractérisé en ce qu'**il comprend les étapes consistant à
a) ajouter du sel phosphinique/de l'acide phosphinique et un oléfine chloré à le solvant et faire réagir avec un oléfine sous l'action d'un amorceur pour obtenir une solution de sel/acide dialkylphosphinique contenant du composé de chlore,
b) faire réagir la solution de sel/acide dialkylphosphinique contenant du composé de chlore avec un composé métallique pour obtenir des sels dialkylphosphiniques contenant du composé de chlore,
où
ledit sel phosphinique est l'hypophosphite de sodium ou l'hypophosphite de potassium,
ledit composé métallique est un composé métallique de Mg, Ca, Al, Zn et Fe,
ledit oléfine chloré est une ou plusieurs substances parmi 1-chloroéthylène, 1,2-dichloroéthylène, 3-chloropropylène, 4-chlorobutylène, ou cyclohexène chloré.

7. Procédé de production de l'additif pour polymères selon la revendication 6, **caractérisé en ce que** ledit solvant est le cyclohexane et/ou le cyclohexène;
ledit amorceur est un amorceur azoïque, un amorceur peroxyde organique ou un amorceur peroxyde inorganique; ledit oléfine est un ou plusieurs mélanges d'éthylène, propylène, butylène, iso-butylène ou pentène.

8. Procédé de production de l'additif pour polymères selon la revendication 1, **caractérisé en ce qu'**il comprend les étapes consistant à
faire réagir une solution de sel dialkylphosphinique avec une solution aqueuse d'un composé métallique contenant l'additif I pour obtenir des sels dialkylphosphiniques correspondants;
où
ledit additif I est une ou plusieurs substances parmi NaCl, KCl, AlCl₃, chlore gazeux, acide chlorhydrique, hypochlorite de sodium, agent de couplage contenant du chlore, tensioactif contenant du chlore;
ledit composé métallique est un composé métallique de Mg, Ca, Al, Zn et Fe;
ledit sel dialkylphosphinique est le dialkylphosphinate de sodium ou le dialkylphosphinate de potassium.

9. Procédé de production de l'additif pour polymères selon la revendication 1, **caractérisé en ce qu'**il comprend les étapes consistant à
ajouter l'additif II pendant une procédure de filtration ou une procédure de lavage ou une procédure de séchage du sel dialkylphosphinique pour obtenir des sels dialkylphosphiniques contenant du composé de chlore;
dans lequel
ledit additif II est une ou plusieurs substances parmi NaCl, KCl, AlCl₃, agent ignifuge contenant du chlore, agent de couplage contenant du chlore, tensioactif contenant du chlore, ou PVC.

10. Utilisation de l'additif pour polymères selon la revendication 1 en tant qu'agent ignifuge.

11. Matériau polymère contenant l'additif selon la revendication 1, comprenant 5-20 parties en poids de l'additif pour polymères, et 50-70 parties en poids des polymères ou leurs mélanges,
dans lequel
ledit additif pour polymères comprend:
A: sels dialkylphosphiniques répondant à la formule (I) 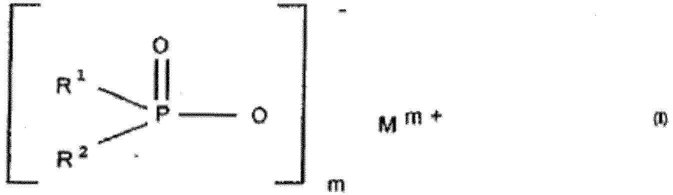 où
R¹, R² sont identiques ou différents et représentent H, C₁-C₆-alkyle ou C₆-C₁₈-aryle;
M est Mg, Ca, Al, Zn, Fe;
m est 2-3;
B: composé de chlore, dans lequel la proportion pondérale de chlore dans les additifs totaux est 50-900 ppm, dans lequel ledit composé de chlore est choisi dans le groupe consistant en une ou plusieurs des substances oléfine chloré, chlorure de sodium, chlorure de potassium, chlorure d'aluminium, chlore gazeux, acide chlorhydrique, hypochlorite de sodium, agent de couplage contenant du chlore, tensioactif contenant du chlore, ou PVC.

12. Matériau polymère selon la revendication 11, **caractérisé en ce que** R¹, R² sont éthyle, propyle, butyle, hexyle ou cyclohexyle.

13. Matériau polymère selon la revendication 11, **caractérisé en ce que** la proportion pondérale dudit chlore dans les additifs totaux est 60-800 ppm, de préférence 70-450 ppm en poids, de préférence encore 100-300 ppm en poids.

14. Matériau polymère selon la revendication 11, **caractérisé en ce que** ledit oléfine chloré est une ou plusieurs substances parmi 1-chloroéthylène, 1,2-dichloroéthylène, 3-chloropropylène, 4-chlorobutylène, ou cyclohexène chloré.

15. Matériau polymère selon la revendication 11, **caractérisé en ce que** ledit polymère est polyester, nylon, PPE, TPE, TPU ou résine époxy, de préférence polyester, nylon ou PPE.

16. Matériau polymère selon la revendication 11, **caractérisé en ce qu'**il comprend en outre 15-30 parties en poids de fibre de verre et 1-5 parties en poids d'autres additifs.
